# EUROPEAN PATENT SPECIFICATION

(11) **EP 1 192 966 B1**
(45) Date of publication and mention of the grant of the patent: **09.11.2005**
(21) Application number: 01308272.2
(22) Date of filing: 27.09.2001
(51) Int. Cl.: A61M 5/31

(54) **Injector with a force sensor on the plunger**
Injektor mit Kraftsensor auf dem Kolben
Injecteur pourvu d'un capteur de force sur son piston

(30) Priority: 28.09.2000 JP 2000297362; 14.02.2001 JP 2001037059
(43) Date of publication of application: 03.04.2002
(73) Proprietor: SEIKAGAKU CORPORATION, Chiyoda-ku, Tokyo 100-0005 (JP)
(72) Inventor: Mitomi, Tokushi, Miura-gun, Kanagawa-ken (JP); Shimojima, Yuji, Higashimurayama-shi, Tokyo (JP)
(74) Representative: Senior, Alan Murray

(56) References cited:
- EP-A- 0 896 827
- FR-A- 2 721 405
- US-A- 4 529 403
- US-A- 5 549 569
- US-A- 5 704 918

## Description

### BACKGROUND OF THE INVENTION

The present invention relates to an injector, and more particularly, to an injector pre-filled with a fluid which may be of high viscoelasticity, which is capable of smoothly pushing out the fluid therefrom without clogging of the fluid at the tip end thereof.

A one chamber-type injector having a syringe previously filled with a fluid and sealed at its tip end by a stopper, also serves as a closed container for storing a fluid therein before its inherent use. Therefore, such an injector enables the fluid to be injected under substantially sterile condition. The one chamber-type injector for usually injecting one kind of fluid, includes a syringe, a plunger inserted into a rear side of the syringe for pushing out the fluid therefrom, and a stopper inserted into a tip end side of the syringe for sealing the fluid. The fluid is filled in the syringe between the plunger and the stopper. Upon use, when the plunger is pushed in the syringe, the stopper is moved together with the fluid toward the tip end of the syringe, so that the fluid is allowed to leak out along an outer peripheral surface of the stopper toward the tip end of the syringe, and then discharge from the syringe through the injection nozzle fitted to the tip end thereof.

In Japanese Utility Model No. 2,580,793, there is described a "pressure holder for an injector having a function as a container" which is a similar type injector to that described above. This injector is provided with bypass grooves which each extend from an inner peripheral surface of a tip end portion of the syringe to an injection nozzle opened to an inner surface of the tip end thereof, in order to allow the fluid to smoothly flow along an outer peripheral surface of the stopper and then discharge therefrom upon push-in operation of a plunger.

Meanwhile, when a high-viscoelastic fluid is filled in the conventional injector having the above structure, it is difficult to push out the fluid through the injection nozzle since clogging of the fluid tends to be caused at a tip end portion thereof. More specifically, in the injector of the above-described type, when the plunger is pushed-in by applying an ordinary pressure thereto, the fluid filled therein reaches the bypass flow path formed on the inner peripheral surface of the tip end portion of the syringe. However, there has been observed such a phenomenon that the fluid cannot flow forwardly from the bypass flow path to the injection nozzle because of its high viscosity.

As a result of the present inventors' earnest studies for developing a syringe structure capable of further reducing a flow resistance of the high-viscoelastic fluid filled therein upon the push-out operation, it has been found that the injector having such a structure can be achieved.

EP-A-0896827, US-A-5704918 and US-A-5549569 also show injectors of this general type.

An object of the present invention is to provide an injector having a syringe previously filled with a high-viscoelastic fluid which is capable of more smoothly pushing out the fluid without clogging at a tip end portion thereof.

In accordance with the present invention, there is provided an injector comprising:
a syringe containing a fluid and having an injection nozzle at a tip end thereof;
a plunger extending into the syringe for pushing the fluid out from the syringe through the injection nozzle;
a stopper for sealing the fluid, located in the syringe between the tip end and the plunger;
the fluid being pre-filled into the syringe between the plunger and the stopper; and
a bypass flow path provided at a tip end portion of the syringe for allowing the fluid to flow therethrough past the outer peripheral surface of the stopper when the stopper is moved to the tip end portion of the syringe by a pushing-in operation of the plunger, characterised in that said syringe is provided on an inner surface of the tip end portion with one or more protrusions to limit movement of the stopper to a position at which there is a clearance, providing a fluid flow path, between the end face of the stopper located at the terminal position and an inner surface of the tip end of the syringe of not greater flow resistance than the flow resistance of the injection nozzle, and wherein the total sectional area of minimum clearances between adjacent ones of the protrusions when viewed in the direction of the inner surface of the tip end of the syringe is not smaller than the opening area of the injection nozzle.

In the injector of the present invention, when the plunger is moved in the pushing-in operation, the stopper is forced, together with the fluid, to move toward the tip end of the syringe. Then, the fluid is allowed to flow through a bypass flow path formed at a tip end portion of the syringe along an outer peripheral surface of the stopper and reach the tip end of the syringe. Further, a plurality of protrusions formed on an inner surface of the tip end of the syringe not only defines a terminal position of movement of the stopper, but also forms a clearance as a fluid flow path at the tip end of the syringe. Preferably, the total sectional area of minimum clearances formed between adjacent ones of the protrusions when viewed in the direction of an inner surface of the tip end of the syringe, is set to the value not smaller than an opening area of the injection nozzle. In such a syringe structure, the flow resistance of the fluid passing through the above flow path is not larger than the flow resistance of the fluid passing through the injection nozzle.

The invention also provides means comprising a syringe, a plunger, and a stopper as described above adapted to be assembled and to contain a fluid so as to form an injector as described above.

### BRIEF DESCRIPTION OF THE DRAWINGS

Fig. 1(a) and Fig. 1(b) are sectional views showing positions of respective components of an injector according to the present invention, taken along a longitudinal center line thereof, before and during a push-out operation, respectively.
Fig. 2(a) to Fig. 2(d) are plan views showing a shape and configuration of protrusions formed on an inner surface of the tip end of a syringe.

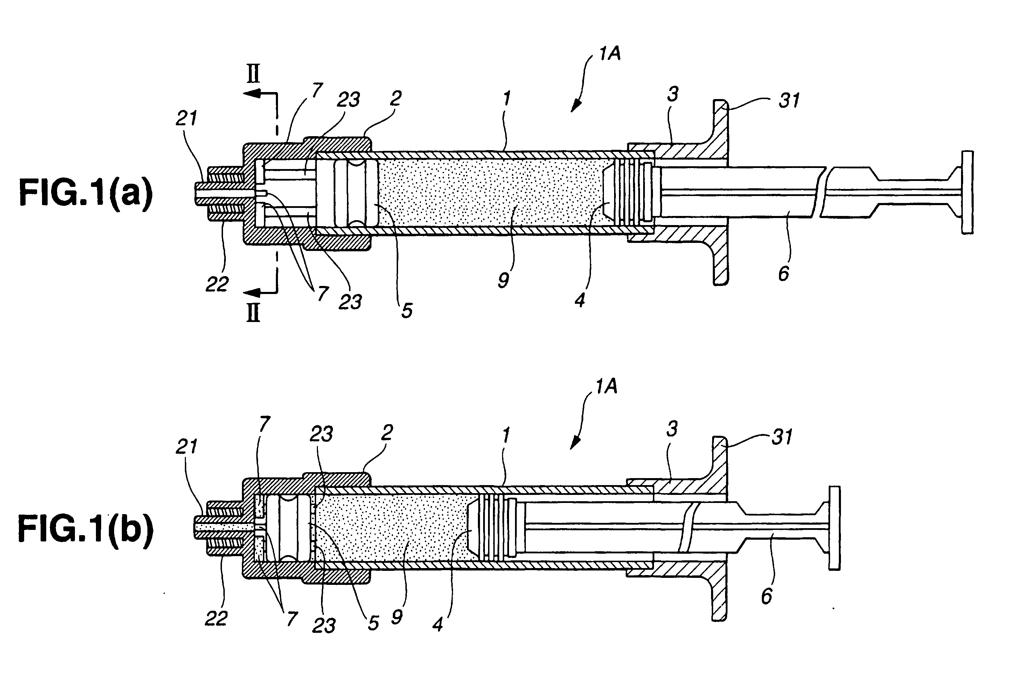

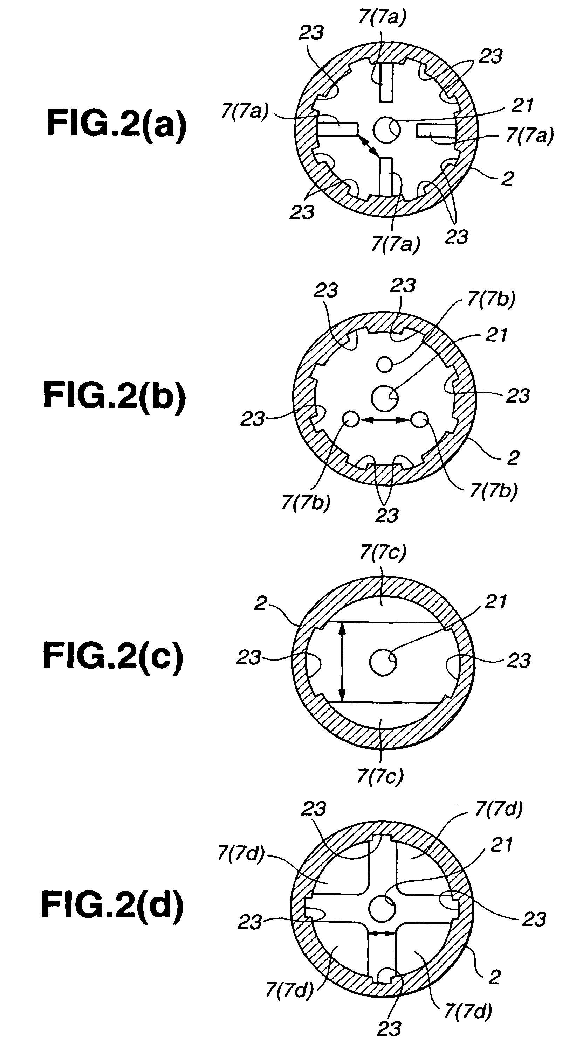

Next, the preferred embodiments of the injector of the present invention will be described in detail by referring to the accompanying drawings. Fig. 1(a) and Fig. 1(b) are sectional views showing a structure of an injector according to the present invention, taken along a longitudinal center line thereof, wherein Fig. 1(a) and Fig. 1(b) show positions of respective components before and during the push-in operation, respectively. Fig. 2(a) to Fig. 2(d) are plan views showing various shapes and configurations of protrusions formed on an inner surface of the tip end of the syringe.

In the injector of the present invention, a syringe thereof is previously filled with a high-viscoelastic fluid. As shown in Fig. 1(a), the injector of the present invention mainly comprises a syringe (1A) provided at a tip end thereof with an injection nozzle (21), a plunger (4) for pushing out a fluid from the syringe, which is inserted into the syringe (1A), a stopper (5) for sealing the fluid which is inserted into the syringe (1A) at a position closer to the tip end than the plunger, a high-viscoelastic fluid (9) previously filled into the syringe between the plunger (4) and the stopper (5), and a bypass flow path such as recesses (23) formed at a tip end portion in the syringe (1A).

The high-viscoelastic fluid (9) filled in the syringe (1A), i.e., a syringe body (1), is not particularly restricted, and any suitable fluid may be used as long as it exhibits a high viscoelasticity. In the present invention, both medical substances (drugs) and non-medical substances may be used as a fluid. Such non-medical substances may include those introduced into human bodies by means of injector or syringe, for example, medical equipments. Specific examples of the medical substances (drugs) may include hyaluronic acid as well as modified products (e.g., cross-linked hyaluronic acid as described in WO97/18244) or gels thereof (hereinafter referred to merely as "hyaluronic acids"), composition of hyaluronic acids and other drugs, collagen, synthetic polymers or the like. The hyaluronic acids may be used, for example, as intra-articular injection agents, auxiliary agents for ophthalmic surgery, adhesion preventives for organs or tissues, etc. in various medical applications. The viscosity of the hyaluronic acids is usually controlled to not less than 5 Pa•s when measured at 25°C using a rotary viscometer. In addition, the high-viscoelastic fluid (9) may include non-medical substances such as adhesives and fillers as long as the substances exhibit a high viscoelasticity.

The syringe (1A) usually comprises a syringe body (1), a end member (2) and a finger-holding member (3). The syringe (1A) shown in Fig. 1 is assembled from respective members produced separately from each other. Alternatively, the respective members may be integrally formed. The syringe body (1) may be of a cylindrical shape and made of a transparent resin material, glass or the like. The thickness of the syringe body may vary depending upon an inner diameter thereof, a pressure applied upon push-out operation, and a contact force of the plunger (4) and the stopper (5), and is so designed as to sufficiently withstand the push-out operation.

The end member (2) is a member for fitting an injection needle (not shown) or the like, if required, to the syringe (1A), and may be usually an injection-molded product made of a synthetic resin having a high chemical resistance and a high heat resistance. Such an end member (2) is of a cylindrical shape having a closed bottom (tip end) which is formed integrally with the injection nozzle (21) and a lure lock (22). The injection nozzle (21) is usually fitted with a tubular injection equipment (not shown) such as an injection needle or a cannula except for the case where the fluid (9) is directly injected from the syringe to aimed sites according to requirements.

A base (rear) portion of the end member (2) has an inner diameter capable of allowing the tip end of the syringe body (1) to be tightly fitted thereinto. Whereas, a tip end portion of the end member (2) has an inner diameter equal to or slightly larger than that of the syringe body (1) as described in detail below. The end member (2) may be usually secured to the syringe body (1) by adhesion using high-frequency welding or adhesive, screwing, interfitting or the like. As a typical bonding structure between the syringe body (1) and the end member (2), there may be appropriately adopted, for example, such a structure in which slightly swelled outer peripheral portions are formed at the tip end of the syringe body (1), and interfitted with grooves formed on an inner peripheral surface of the base portion of the end member (2).

In the preferred embodiment of the present invention, the end member (2) may be provided with the lure lock (22) which serves as a mechanism for preventing falling-off of the injection needle fitted onto the injection nozzle (21). The lure lock (22) is of a cylindrical structure surrounding the injection nozzle (21), and is provided on an inner peripheral surface thereof with threads or grooves which are engaged with an outwardly extending flange formed on a base of the injection needle. Meanwhile, in order to prevent the falling-off of the injection needle, there may be used other mechanisms having a similar function to that of the lure lock. Also, in the case where the injection needle is formed integrally with the injection nozzle (21), the mechanism for preventing the falling-off of the injection needle may be omitted.

The finger-holding member (3) may be usually of a substantially cylindrical shape having two ear-shaped hooks (31) outwardly extending in opposite directions from a rear end thereof. The finger-holding member (3) is interfitted to a rear side of the syringe body (1) by inserting the rear end of the syringe body thereinto. Meanwhile, the finger-holding member (3) may be fitted to the syringe body at a position closer to the tip end thereof when the whole length of the syringe body is small. Such a finger-holding member (3) may be usually secured to the syringe body (1) by adhesion using high-frequency welding or adhesive.

The plunger (4) may be usually made of an elastic material such as rubbers and synthetic rubbers so as to closely contact with the inner peripheral surface of the syringe body (1). The plunger (4) has, for example, a cylindrical body on an outer peripheral surface of which three ring-shaped packings are arranged along the center line direction in order to improve its air-tightness to the syringe body (1). The plunger (4) is provided at its rear end with a plunger rod (6) for the push-in operation thereof. Meanwhile, in Fig. 1(a), there is shown the plunger (4) inserted into the rear end of the syringe (1A). However, the plunger may be inserted to an intermediate portion of the syringe (1A) when the amount of the fluid filled therein is smaller.

The stopper (5) serves as a sealing member for preventing leakage of the fluid (9) previously filled in the syringe body (1), and may be made of an elastic material such as rubbers and synthetic rubbers similarly to the plunger (4). The stopper (5) has any suitable outer shape capable of being closely fitted with an inner peripheral surface of the syringe body (1), for example, a short-cylindrical shape having a slightly larger diameter at tip and rear ends thereof.

As described hereinafter, the stopper (5) is moved toward the tip end of the syringe (1A), i.e., toward the position where the end member (2) is fitted to the syringe, upon the push-in operation of the plunger (4). The end member (2) is provided in its inner peripheral surface with a bypass flow path such as recesses (23) through which the fluid (9) is flowed out along the outer peripheral surface of the stopper (5) which has now been moved to the terminal position by the push-in operation of the plunger (4). As shown in Fig. 1(a) and Fig. 2(a) to Fig. 2(d), the recesses (23) are in the form of a plurality of grooves formed on the inner peripheral surface of the end member (2) along the center line thereof for defining a flow path of the fluid (9) between the inner peripheral surface of the end member (2) and the outer peripheral surface of the stopper (5).

In addition to the recesses (23) as shown above, the bypass flow path may have, for example, an alternative structure in which the end member (2) has a larger inner diameter than that of the syringe body (1) at its tip end portion so as to form a constant clearance between the whole inner peripheral surface of the end member (2) and a whole outer peripheral surface of the stopper (5), thereby enabling the fluid (9) to flow out along the whole outer peripheral surface of the stopper (5). In any structure, the total sectional area of the bypass flow path is preferably larger than the opening area of the injection nozzle (21) in order to reduce the flow resistance of the fluid passing therethrough upon the push-out operation, and allow the fluid (9) to more smoothly flow toward the injection nozzle (21).

Here, the "total sectional area of the bypass flow path" means the sum of sectional areas of individual clearances formed between the whole inner peripheral surface of the tip end portion of the end member (2) and the whole outer peripheral surface of the stopper (5) when viewed in the direction from the rear side of the syringe (1A) (i.e., the side where the plunger (4) is located) toward the tip end thereof (i.e., the side where the injection nozzle (21) is located). Therefore, when the inner diameter of the tip end of the end member (2) is identical to that of the syringe body (1), the "total sectional area of the bypass flow path" is the sum of sectional areas of the recesses (23). For example, in the structure as shown in Fig. 2(a) and Fig. 2(b) in which the recesses (23) are constituted by eight small rectangular spaces formed on the inner peripheral surface of the end member (2), the total sectional area of the bypass flow path is the sum of sectional areas of the eight small rectangular spaces. In the structure as shown in Fig. 2(c) in which the recesses (23) are constituted by two opposed wide spaces of a substantially rectangular shape formed along an circular contour of the inner peripheral surface of the end member (2), the total sectional area of the bypass flow path is the sum of sectional areas of the two opposed spaces. In addition, in the structure as shown in Fig. 2(d) in which the recesses (23) are constituted by four small rectangular spaces formed on the inner peripheral surface of the end member (2), the total sectional area of the bypass flow path is the sum of sectional areas of the four small rectangular spaces. On the other hand, when the inner diameter of the tip end portion of the end member (2) is larger than that of the syringe body (1), the "total sectional area of the bypass flow path" is the sum of sectional areas of the recesses (23) plus sectional areas of clearances formed between the whole outer peripheral surface of the stopper (5) and the whole inner peripheral surface of the tip end portion of the end member (2). Meanwhile, the "opening area of the injection nozzle (21)" means a sectional area of an opening provided on the center of the inside tip end face of the end member (2) as shown in Fig. 2(a) to Fig. 2(d).

In the injector of the present invention, in order to prevent the tip end of the syringe (1A) from being clogged with the high-viscoelastic fluid (9) such as hyaluronic acids, a plurality of protrusions (7) are formed on the inner surface of the tip end of the syringe (1A) where the injection nozzle is opened, i.e., on the inner surface of the tip end of the end member (2) so as to define a terminal position of movement of the stopper (5) and form a clearance as a fluid flow path between the tip end face of the stopper (5) and the inner surface of the tip end of the end member (2) when the stopper (5) is moved to the terminal position.

The protrusions (7) are usually integrally formed on the inner surface of the tip end of the end member (2). As far as the protrusions (7) each have shape and size so as not to be buried in the stopper due to deformation of the elastic stopper (5) when pressing the stopper (5) made of the elastic material, and are capable of maintaining a sufficient clearance between the tip end face of the stopper (5) and the inner surface of the tip end of the end member (2), the protrusions (7) each may have any suitable shape and size and an appropriate number of the protrusions may be formed. For example, the protrusions (7) may be so designed as to have various shapes as shown by reference numerals (7a), (7b), (7c) and (7d) in Fig. 2(a) to Fig. 2(d).

In Fig. 2(a), there are shown four rib-like protrusions (7a) extending radially outwardly from the central opening of the injection nozzle (21) in the respective four directions. The four protrusions are formed at positions where the outer ends thereof do not overlap the recesses (23). Further, the ends of the protrusions 7(a), turned against the opening of the injection nozzle (21), are located slightly apart from the opening of the injection nozzle (21) in order to ensure smooth flow of the fluid (9) to the injection nozzle (21). Such protrusions (7a) are advantageous from the standpoint of accurately determining the terminal position of the stopper (5).

In Fig. 2(b), there are shown three cylindrical protrusions (7b) disposed equidistantly around the opening of the injection nozzle (21). Such protrusions (7b) are advantageous from the standpoint of further reducing a flow resistance of the fluid (9) flowing along the inner surface of the tip end of the end member (2), and being readily formed by molding.

Further, in Fig. 2(c), there are shown two opposed protrusions (7c) formed by swelling a part of the inner surface of the tip end of the end member (2) toward the tip end of the syringe body (1), i.e., toward the rear side of the end member. More specifically, the protrusions (7c) have such a shape that two opposed portions of the inner surface of the tip end of the end member (2) corresponding to circular portions each having a constant peripheral length are raised in a step-like manner. As shown in Fig. 2(c), the protrusions (7c) are formed so as not to overlap the wide recesses (23) formed on the inner peripheral surface of the end member (2). Such protrusions (7c) are advantageous from the standpoint of accurately defining the terminal position of the stopper (5), and being readily formed by molding.

Also, in Fig. 2(d), there are shown four flat protrusions (7d) each having a substantially triangular shape which are disposed equidistantly apart from each other in respective four directions around the opening of the injection nozzle (21). The protrusions (7d) are formed by swelling parts of the inner surface of the tip end of the end member (2) toward the tip end of the syringe body (1), i.e., toward the rear side of the end member. Further, the vertex of the triangular shape of the respective four protrusions (7d), turned against the opening of the injection nozzle (21), is located slightly apart from the opening of the injection nozzle (21) in order to smoothly spread the fluid (9) in the direction of the inner surface of the tip end of the end member (2) around the opening of the injection nozzle (21), thereby ensuring smooth flow of the fluid (9) toward the injection nozzle (21). As shown in Fig. 2(d), the protrusions (7d) are formed so as not to overlap the recesses (23), namely, the recesses (23) are formed at positions corresponding to grooves between the adjacent protrusions (7d, 7d). Such protrusions (7d) are advantageous from the standpoint of accurately defining the terminal position of the stopper (5), being readily formed by molding, and ensuring the smooth flow of the fluid (9).

In the present invention, in order to surely reduce the flow resistance of the fluid (9) upon the push-out operation thereof, the total sectional area of minimum clearances formed between the adjacent protrusions (7, 7) when viewed in the direction of the inner surface of the tip end of the end member (2), is set to the value not smaller than the opening area of the injection nozzle (21) on the inner surface of the tip end of the syringe (1A). Such a structure enables the fluid (9) to be smoothly supplied from the bypass flow path toward the injection nozzle (21).

More specifically, the minimum clearances between the adjacent protrusions (7, 7) are spaces as shown by the arrows in Fig. 2(a) to Fig. 2(d). The shape, height and configuration of the protrusions (7) are appropriately determined such that the total sectional area of such minimum clearances (sectional areas in the direction indicated by each arrow and perpendicular to the paper surface), i.e., total sectional area of four clearances in Fig. 2(a), total sectional area of three clearances in Fig. 2(b), total sectional area of one clearance in Fig. 2(c) and total sectional area of four clearances in Fig. 2(d), is not smaller than the opening area of the injection nozzle (21).

Referring to Fig. 1(a), Fig. 1(b) and Fig. 2(a), the "total sectional area of minimum clearances between the protrusions (7, 7)" will be described in more detail below. That is, when the stopper (5) is moved to the terminal position as shown in Fig. 1(b), there are formed four spaces surrounded by the tip end face of the stopper (5), the inner surface of the tip end of the end member (2) and the four protrusions (7a). In each of the spaces, the minimum clearance means a section perpendicular to the direction from the inner peripheral surface of the end member (2) toward the opening of the injection nozzle (21) (i.e., the radial direction of the inner surface of the tip end of the end member (2)). The sum of the minimum sectional areas between the adjacent protrusions is the "total sectional area of minimum clearances between the protrusions (7, 7)".

In the injector of the present invention, the injection nozzle (21) of the end member (2) may be fitted with an injection needle or the like upon practical use. In this case, the injection needle may be secured to a base of the injection nozzle through the lure rock (22), for example, by rotating the injection needle at an angle of about 90°. When the fluid (9) is injected from the injector, fingers are placed on the opposite hooks (31) of the finger-holding member (3), and then a pressing force is applied to the rear end of the plunger rod (6) in order to push-in the plunger (4) into the syringe.

When the plunger (4) is pushed-in by the pressing operation of the plunger rod (6), the fluid (9) is moved together with the stopper (5) toward the tip end of the syringe (1A) by the pressing force applied. When the stopper (5) reaches its terminal position at the tip end of the end member (2), the recesses (23) formed on the inner peripheral surface of the end member (2) are opened to the outer peripheral surface of the stopper (5), thereby forming a flow path communicating with the inside of the syringe body (1). As a result, the fluid (9) filled in the syringe body (1) is allowed to flow along the outer peripheral surface of the stopper (5) toward the tip end of the end member (2).

Also, the plurality of the protrusions (7) provided on the inner surface of the tip end of the end member (2) can not only surely define the terminal position of the stopper (5), but also forms clearances serving as a fluid flow path at the inner tip end of the end member (2). In this case, with such a structure in which the total sectional area of minimum clearances between the adjacent protrusions (7, 7) is not smaller than the opening area of the injection nozzle (21), the flow resistance of the fluid (9) passing through the clearances formed at the tip end of the end member (2) always becomes smaller than that of the fluid passing through the injection nozzle (21).

As a result, in the injector of the present invention, it is possible to effectively prevent the inner tip end of the end member (2) from being clogged with the fluid (9) flowing therethrough. In other words, the fluid (9) is free from clogging on the inner surface of the tip end of the end member (2), i.e., at the tip end of the syringe (1A), so that it becomes possible to smoothly push out the high-viscoelastic fluid such as hyaluronic acids through the injection nozzle (21), and inject the fluid to aimed sites.

Further, in the injector of the present invention, since the total sectional area of the bypass flow path is set to the value not smaller than the opening area of the injection nozzle (21) as described above, the flow resistance of the fluid (9) passing through the bypass flow path upon the push-out operation can be adequately reduced, so that it becomes possible to more smoothly push out the fluid (9) from the injector.

Thus, as discussed above, in the injector of the present invention, specific protrusions are formed so as to provide clearances as a fluid flow path at the tip end of the syringe, and the flow resistance of the fluid passing through the clearances always becomes smaller than that of the fluid passing through the injection nozzle. Therefore, it is possible to prevent the tip end of the syringe from being clogged with the fluid, and smoothly push out the high-viscoelastic fluid through the injection nozzle.

## Claims

1. An injector comprising:
a syringe (1A) containing a fluid (9) and having an injection nozzle (21) at a tip end thereof;
a plunger (4) extending into the syringe for pushing the fluid out from the syringe through the injection nozzle (21);
a stopper (5) for sealing the fluid, located in the syringe (1A) between the tip end and the plunger;
the fluid (9) being pre-filled into the syringe between the plunger (4) and the stopper (5); and
a bypass flow path provided at a tip end portion of the syringe (1A) for allowing the fluid (9) to flow therethrough past the outer peripheral surface of the stopper (5) when the stopper is moved to the tip end portion of the syringe by a pushing-in operation of the plunger (4), **characterised in that** said syringe is provided on an inner surface of the tip end portion with one or more protrusions (7) to limit movement of the stopper (5) to a position at which there is a clearance, providing a fluid flow path, between the end face of the stopper (5) located at the terminal position and an inner surface of the tip end of the syringe (1A) of not greater flow resistance than the flow resistance of the injection nozzle, and wherein the total sectional area of minimum clearances between adjacent ones of the protrusions (7, 7) when viewed in the direction of the inner surface of the tip end of the syringe (1A) is not smaller than the opening area of the injection nozzle (21).

2. An injector according to claim 1, wherein the total sectional area of the bypass flow path is not smaller than the opening area of the injection nozzle (21).

3. An injector according to claim 1 or 2, wherein the high-viscoelastic fluid is a hyaluronic acid product.

4. An injector according to any preceding claim, wherein the tip end portion of the syringe (1A) is a part made separately from the body of the syringe.

5. An injector according to any preceding claim, wherein the bypass flow path comprises recesses (23) in the inner wall of the tip end portion.

6. An injector according to any one of claims 1 to 4, wherein the bypass flow path is created by the diameter of the tip end portion being larger than that of the stopper.

7. An injector according to any preceding claim, wherein the protrusions (7) are integral with the tip end portion.

8. An injector according to any preceding claim, wherein the fluid is of high viscoelasticity.

## Patentansprüche

1. Injektor umfassend:
Eine Spritze (1A) beinhaltend ein Fluid (9)und aufweisend eine Injektionsdüse (21) an ihrem Düsenende;
einen Kolben (4), der sich in die Spritze hineinerstreckt zum Ausstoßen des Fluids aus der Spritze heraus durch die Injektionsdüse (21);
einen Pfropfen (5) zum Abdichten des Fluids, der in der Spritze (1A) zwischen dem Düsenende und dem Kolben angeordnet ist;
wobei das Fluid (9) zwischen dem Kolben (4) und den Pfropfen (5) in die Spritze voreingefüllt ist; und
einen Bypass-Strömungsweg, der in einem Düsenendbereich der Spritze (1A) vorgesehen ist, um es dem Fluid (9) zu ermöglichen, hindurchzufließen an der äußeren Umfangsoberfläche des Pfropfens (5) wobei wenn der Pfropfen in den Düsenbereich der Spritze durch einen Einschubvorgang des Kolbens (4) bewegt wird, **dadurch gekennzeichnet, dass** die Spritze an einer inneren Oberfläche des Düsenbereichs mit einem oder mehreren Vorsprüngen (7) versehen ist, um die Bewegung des Pfropfens (5) auf eine Position zu beschränken, an der ein Abstand besteht, der einen Fluid-Strömungsweg zwischen der Endoberfläche des Pfropfens (5), der in der Endposition angeordnet ist und einer inneren Oberfläche des Düsenendes der Spritze (1A) bereitstellt von einem nicht größeren Strömungswiderstand, als dem Strömungswiderstand der Injektionsdüse und wobei die totale Durchschnittsfläche minimaler Abstände zwischen den gegenüberliegenden Ausstülpungen (7, 7), wenn sie in der Richtung der inneren Oberfläche des Düsenendes der Spritze (1A) betrachtet werden, nicht kleiner ist, als die Öffnungsfläche der Injektionsdüse (21).

2. Injektor gemäß Anspruch 1, wobei die totale Durchschnittsfläche des Bypass-Strömungsweges nicht kleiner ist, als die Öffnungsfläche der Injektionsdüse (21).

3. Injektor gemäß Anspruch 1 oder 2, wobei das hoch viskoelastische Fluid ein Hyaluronsäureprodukt ist.

4. Injektor gemäß einem der vorstehenden Ansprüche, wobei der Düsenendbereich der Spritze (1A) ein Teil ist, das separat von dem Körper der Spitze ausgebildet wurde.

5. Injektor gemäß einem der vorstehenden Ansprüche, wobei der Bypass-Strömungspfad Ausnehmungen (23) in der inneren Wand des Düsenendbereichs umfasst.

6. Injektor gemäß einem der Ansprüche 1 bis 4, wobei der Bypass-Strömungskanal **dadurch** erzeugt wird, dass der Durchmesser des Düsenendbereichs größer ist, als der des Pfropfens.

7. Injektor gemäß einem der vorstehenden Ansprüche, wobei die Vorsprünge (7) fest mit dem vorderen Endbereich verbunden sind.

8. Injektor gemäß einem der vorstehenden Ansprüche, wobei das Fluid eine hohe Viskoelastizität aufweist.

## Revendications

1. Injecteur comprenant :
une seringue (1A) contenant un fluide (9) et comportant une buse d'injection (21) au niveau de l'extrémité formant bout de celle-ci ;
un piston (4) s'étendant dans la seringue afin de pousser le fluide hors de la seringue à travers la buse d'injection (21) ;
un bouchon (5) destiné à sceller le fluide, situé dans la seringue (1A) entre l'extrémité formant bout et le piston ;
le fluide (9) étant rempli au préalable dans la seringue entre le piston (4) et le bouchon (5) ; et
un circuit de dérivation d'écoulement prévu au niveau de la partie d'extrémité formant bout de la seringue (1A) afin de permettre au fluide (9) de s'écouler à travers celui-ci au-delà de la surface périphérique extérieure du bouchon (5) lorsque le bouchon est déplacé vers la partie d'extrémité formant bout de la seringue par une opération de poussée du piston (4), **caractérisé en ce que** ladite seringue est munie, sur une surface intérieure de la partie d'extrémité formant bout, d'une ou de plusieurs saillies (7) servant à limiter le mouvement du bouchon (5) dans une position dans laquelle il existe un espace, fournissant un circuit d'écoulement de fluide, entre la face d'extrémité du bouchon (5) située dans la position terminale et une surface intérieure de l'extrémité formant bout de la seringue (1A) dont la résistance à l'écoulement n'est pas supérieure à la résistance à l'écoulement de la buse d'injection, et dans lequel la surface totale d'espaces minimaux entre les saillies adjacentes (7, 7) vue dans la direction de la surface intérieure de l'extrémité formant bout de la seringue (1A) n'est pas inférieure à la surface d'ouverture de la buse d'injection (21).

2. Injecteur selon la revendication 1, dans lequel la surface totale du circuit de dérivation d'écoulement n'est pas inférieure à la surface d'ouverture de la buse d'injection (21).

3. Injecteur selon la revendication 1 ou 2, dans lequel le fluide à forte viscosité élastique est un produit d'acide hyaluronique.

4. Injecteur selon l'une quelconque des revendications précédentes, dans lequel la partie d'extrémité formant bout de la seringue (1A) est une partie réalisée séparément du corps de la seringue.

5. Injecteur selon l'une quelconque des revendications précédentes, dans lequel le circuit de dérivation d'écoulement comprend des évidements (23) dans la paroi intérieure de la partie d'extrémité formant bout.

6. Injecteur selon l'une quelconque des revendications 1 à 4, dans lequel le circuit de dérivation d'écoulement est créé par le fait que le diamètre de la partie d'extrémité formant bout est plus grand que celui du bouchon.

7. Injecteur selon l'une quelconque des revendications précédentes, dans lequel les saillies (7) font corps avec la partie d'extrémité formant bout.

8. Injecteur selon l'une quelconque des revendications précédentes, dans lequel le fluide a une forte viscosité élastique.
